# EUROPÄISCHE PATENTANMELDUNG

(11) **EP 0 635 256 A1**
(43) Veröffentlichungstag der Anmeldung: **25.01.1995**
(21) Anmeldenummer: 94109335.3
(22) Anmeldetag: 17.06.1994
(51) Int. Cl.: A61K 6/06, A61K 6/02

(54) **Verfahren zur Herstellung oxidkeramischer Zahnersatzstücke**

(30) Priorität: 21.07.1993 DE 4324438
(71) Anmelder: Degussa Aktiengesellschaft, D-60311 Frankfurt (DE)
(72) Erfinder: Beyer, Hans-Hermann, Dr. Dipl.-Chem., D-83796 Kahl/Main (DE); Biberbach, Peter, Dipl.-Ing., D-63517 Rodenbach (DE)

(57) **Zusammenfassung**

Zur Herstellung paßgenauer oxidkeramischer Zahnersatzstücke in einem Arbeitsgang muß ein Schrumpfen des Gemisches aus Keramikteilchen mit Bindemitteln und/oder Anmischflüssigkeiten beim Sintern verhindert werden. Das erreicht man durch Zugabe von Teilchen aus leicht oxidierbaren Metallen, Metallsuboxiden und/oder Metallhydriden, die beim Sinter unter Sauerstoffaufnahme eine Volumenvergrößerung erfahren und die Sinterschrumpfung bei entsprechendem Mischungsverhältnis kompensieren.

## Beschreibung

Die Erfindung betrifft ein Verfahren zur Herstellung oxidkeramischer Zahnersatzstücke, wie Inlays, Kronen oder sonstiger Prothesenteile, in einem Arbeitsgang, durch Sintern eines Gemisches aus Oxidkeramikteilchen mit Bindemitteln und/oder Anmischflüssigkeiten.

In der Zahnheilkunde stehen eine größere Anzahl von Materialien für Füllungen und Prothesenteilen zur Verfügung. Amalgame haben als Füllungsmaterialien eine weite Verbreitung, da sie eine gute Verarbeitbarkeit und ein günstiges Abrasionsverhalten im Molarenbereich besitzen. Nachteilig ist jedoch die Färbung, die nicht der Zahnfarbe entspricht. Außerdem sind die Amalgame wegen ihres Quecksilbergehaltes in Verruf gekommen.

Die sogenannten Composites sind aufwendiger in der Verarbeitung, die Haltbarkeit ist begrenzt, wobei über das Langzeitverhalten noch wenig Erfahrung vorliegen. Sie werden überwiegend im Frontzahnbereich eingesetzt, da die Zahnfarbe sich sehr gut einstellen lässt.

Goldgußfüllungen und sonstige Goldgußteile sind von der Herstellung recht aufwendig, die Materialkosten liegen sehr hoch. Störend wirkt oft die Metallfarbe, obwohl teilweise Keramikverblendungen möglich sind.

Keramikinlays erfüllen viele Anforderungen bezüglich Haltbarkeit und Farbgebung. Ihre Herstellung ist jedoch meist aufwendig, was auch für die Herstellung von Kronen und sonstiger prothetischer Teile gilt. Üblicherweise werden die keramischen Massen, bestehend aus oxidkeramischen Pulvern, Bindern und Anmischflüssigkeiten, auf einer feuerfesten Stumpfmasse zur Erzeugung der notwendigen Festigkeit bei Temperaturen von 1100 bis 1200^{o} C gesintert. Zum Ausgleich der hierbei auftretenden Sinterschrumpfung und der damit verbundenen Paßungenauigkeit werden die keramischen Massen schichtweise in mehreren Brennvorgängen aufgesintert. Durch eine genaue Abstimmung der Stumpfmasse und der aufzusinternden Keramik wird versucht, die durch Schrumpfung auftretenden Maßänderungen zu minimieren. Nach dem Brennvorgang ist bei diesem Verfahren eine mechanische Entfernung der Stumpfmasse erforderlich, wodurch die Sinterkörper beschädigt werden können.

So werden beispielsweise in der EP-OS 240 643 zahntechnische Prothesenteile durch schichtweisen Aufbau aus einem Keramikschlicker mit Wasser auf einem Stumpfmodell hergestellt. Die Schichten werden bei etwa 1100^{o} C gesintert, ohne daß eine wesentliche Schrumpfung stattfindet. Die noch vorhandenen Poren werden mit einer Glasmasse ausgefüllt.

Aus der EP-PS 214 341 ist die Herstellung metallischer Prothesenteile bekannt, wobei die dort verwendeten Edelmetalle Keramik und Glas enthalten können. Es wird mit Wasser ein Schlicker angerührt und die damit erzeugten Grünlinge gesintert. Durch die Auswahl bestimmter Edelmetallpulverteilchengrößen wird erreicht, daß die Schrumpfung beim Sintern minimal ist. Keramische Teile lassen sich auf diese Weise nicht herstellen.

Es war daher Aufgabe der vorliegenden Erfindung, ein Verfahren zur Herstellung oxidkeramischer Zahnersatzstücke, wie Inlays, Kronen oder sonstiger Prothesenteile, durch Sintern eines Gemisches aus Oxidkeramikteilchen, Bindemitteln und/oder Anmischflüssigkeiten zu entwickeln, das in einem Arbeitsgang zu paßgenauen Teilen führt und ohne Verwendung von Stumpfmodellen auskommt.

Diese Aufgabe wird erfindungsgemäß dadurch gelöst, daß den Keramikteilchen Teilchen aus leicht oxidierbaren Metallen, Metallsuboxiden und/oder Metallhydriden zugesetzt werden, wobei deren stabilen Oxide eine zahnähnliche Färbung aufweisen müssen,
daß aus dem Gemisch Grünlinge mit den gewünschten Enddimensionen geformt werden,
daß das Sintern unter Luft- bzw.- Sauerstoffzufuhr erfolgt, und
daß das Gewichtsverhältnis von Metall-, Metallsuboxid- und/oder Metallhydridteilchen zu Keramikteilchen so gewählt wird, daß die Volumenschrumpfung der Keramikteilchen beim Sintern durch die Volumenvergrößerung der Metall-, Metallsuboxid und/oder Metallhydridteilchen bei der Oxidation kompensiert wird.

Vorzugsweise verwendet man als Metallteilchen Pulver aus Titan, Zirkonium oder Aluminium. Die Oxide dieser Metalle sind weiß bzw. besitzen eine zahnähnliche Färbung. Als Metallsuboxide können beispielsweise Titandioxid oder Zirkoniumdioxid, als Metallhydride beispielsweise Titanhydrid oder Zirkoniumhydrid dienen, deren Oxide ebenfalls weiß bzw. zahnfarben sind. Es sind nur solche Metalle bzw. Suboxide oder Metallhydride einsetzbar, die bei der Sintertemperatur von ca. 1300^{o} C an Luft bzw. Sauerstoffoxiden und unter Volumenvergrößerungen stabile Oxide bilden. Das erforderliche Gewichtsverhältnis von Metallteilchen zu Keramikteilchen muß durch Vorversuche ermittelt werden.

Die verwendeten Bindersysteme und/oder Anmischflüssigkeiten dienen hauptsächlich der Formgebung der Grünlinge, sie können jedoch auch, falls sie anorganische Komponenten enthalten, Bestandteile der Keramik werden. Vorzugsweise verwendet man als Binder Wachse und Methacrylate, die rückstandsfrei ausbrennen, oder Silikone, die beim Erhitzen Siliziumdioxid bilden, das von der Keramik aufgenommen wird, oder anorganische Phosphate, die als Sinterhilfsmittel dienen.

Als Anmischflüssigkeiten dienen vorzugsweise Wasser und Polyalkohole, als keramische Füllstoffe vor allem Metalloxide, Siliziumoxid und/oder Silikate.

Man erhält mit diesem Verfahren oxidkeramische Zahnersatzstücke hoher Paßgenauigkeit mit relativ geringem Arbeitsaufwand, verglichen mit den bisher bekannten Verfahren. Die so hergestellten Teile sind dichtgesintert und besitzen dadurch eine hohe mechanische Festigkeit. Sie zeigen eine zahnähnliche Farbe, können jedoch zur Verbesserung der ästhetischen Wirkung noch einem Farb- und Glanzbrand unterworfen werden.

Das Prinzip des erfindungsgemäßen Verfahrens wird im folgenden am Beispiel des Titans beschrieben, ist jedoch in gleicher Weise für die anderen genannten Metalle und/oder ihre Verbindungen gültig.

Titanpulver reagiert beim Erhitzen an Luft bereits bei relativ niedrigen Temperaturen mit Sauerstoff unter Bildung von Titanoxid. Der Oxidationsvorgang kann hierbei so gesteuert werden, daß eine allmähliche Sauerstoffaufnahme erfolgt und eine Überhitzung durch die ansonsten stark exotherme Reaktion vermieden wird. Der Übergang ins Oxid ist aufgrund der Massenzunahme und der unterschiedlichen Dichten von Titan und Titanoxid mit einer erheblichen Volumenzunahme von ca. 78,3 % verbunden. Bei einem porösen Formkörper, werden hierbei die inneren Poren weitgehend geschlossen. Der Formkörper kann durch weiteres Erhitzen noch verdichtet werden. Die übliche Sinterschrumpfung wird durch die Oxidbildung kompensiert. Durch eine auf die zu erwartende Schrumpfung abgestimmte Mischung von Titanpulver und Metalloxiden,
wie Ti0₂, Zr0₂, Erdalkalioxiden oder Verbindungen dieser Oxide, kann somit ein dicht gesinterter Formkörper mit hoher Festigkeit und zahnfarbener Farbe gefertigt werden, der nach dem Sinterprozeß exakt die gleichen Dimensionen besitzt wie der Grünling.

Folgende Beispiele sollen das erfindungsgemäße Verfahren näher erläutern:
1. In einem Kneter werden folgende Rohstoffe bei ca. 160^{o} C homogen zu einer plastischen Masse vermischt:
   36,4 % Titanpulver, mittlere Teilchengröße 7µm;
   27,3 % Magnesiumtitanat, mittlere Teilchengröße 2µm;
   27,3 % Zirkonoxid, mittlere Teilchengröße 1µm;
   9,0 % Wachs.
   Aus der so hergestellten, bei höherer Temperatur plastischen Masse, wird unter Zuhilfenahme von geeigneten Formen der gewünschte Formkörper z.B. Inlays, Onlays, Kronen, als Grünling hergestellt. In einem programmgesteuerten Brennprozess wird zunächst mit einer Aufheizrate von ca. 0,5^{o} C/min bis zu einer Temperatur von 370^{o} C das Wachs ausgebrannt. Es entsteht ein formstabiler Rohling mit einer Gründichte von ca. 70 Vol. %. Beim weiteren Erhitzen mit einer Aufheizrate von ca. 2^{o} C/min auf ca. 1300^{o} C an Luft werden die noch vorhandenen Poren durch das sich bildende Titanoxid allmählich geschlossen. Bei einer
   Temperatur von ca. 1300^{o} C wird während 3 Stunden der Formkörper auf sein Enddichte gesintert. Nach Abkühlen auf Raumtemperatur besitzt das Sinterprodukt exakt die Ausgangsdimensionen des Formkörpers.
2. In einem Kneter werden folgende Rohstoffe homogen zu einer plastischen Masse vermischt:
   56 % Titanpulver, mittlere Teilchengröße 7µm;
   13 % Titandioxid, mittlere Teilchengröße 2µm;
   10 % Magnesiumoxid, mittlere Teilchengröße 8µm;
   21 % Methacrylat.
   Aus der so hergestellten Masse wird unter Zuhilfenahme von geeigneten Formen der gewünschte Formkörper z.B. Inlays, Onlays, Kronen, hergestellt.
   Nach dem Aushärten des Binders wird in einem programmgesteuerten Brennprozess analog Beispiel 1 zunächst das Methacrylat ausgebrannt. Es entsteht ein formstabiler Rohling mit einer Gründichte von ca. 68 Vol. %. Beim weiteren Erhitzen werden die noch vorhandenen Poren durch das sich bildende Titanoxid allmählich geschlossen. Bei einer Temperatur von ca. 1300^{o} C wird der Formkörper während 3 Stunden auf seine Enddichte gesintert. Nach Abkühlen auf Raumtemperatur besitzt das Sinterprodukt exakt die Ausgangsdimensionen des Formkörpers.
3. Auf einer Glasplatte wird eine Pulvermischung aus 50 % Titanpulver, 33 % Magnesiumoxid und 17 % Ammoniumdihydrogenphosphat mit Wasser zu einer homogenen Paste vermischt und unter Zuhilfenahme von geeigneten Formen der gewünschten Formkörper hergestellt.
   Nach dem Trocknen und Abbinden wird in einem programmgesteuerten Brennprozess mit einer Aufheizrate von ca. 2^{o} C/min gebrannt, wobei zunächst Ammoniak und das beim Abbindeprozeß eingebaute Wasser entweicht. Es entsteht ein formstabiler Rohling mit einer Gründichte von ca. 70 %. Beim weiteren Erhitzen werden die noch vorhandenen Poren durch das sich bildende Titanoxid allmählich geschlossen. Bei einer Temperatur von ca. 1300^{o} C wird der Formkörper auf seine Enddichte gesintert. Nach Abkühlen auf Raumtemperatur besitzt das Sinterprodukt exakt die Ausgangsdimensionen des Formkörpers.
4. Auf einer Glasplatte wird eine Pulvermischung aus 50 % Titanpulver, 37 % Magnesiumoxid und 13 % Titanoxid mit Wasser und Polyäthylenglykol zu einer homogenen Paste vermischt und unter Zuhilfenahme von geeigneten Formen der gewünschte Formkörper hergestellt. Nach dem Trocknen und Abbinden wird in einem programmgesteuerten Brennprozess mit einer Aufheizrate von 2^{o} C/min gebrannt, wobei zunächst das beim Abbbindeprozeß eingebaute Wasser entweicht. Im weiteren Verlauf wird das Plyäthylenglykol oxidiert und die hierdurch gebildeten Gase ausgetrieben. Es entsteht ein formstabiler Rohling mit einer Gründichte von ca. 70 %. Beim weiteren Erhitzen werden die noch vorhandenen Poren durch das sich bildende Titanoxid geschlossen. Bei einer Temperatur von ca. 1300^{o} C wird der Formkörper auf seine Enddichte gesintert. Nach Abkühlen auf Raumtemperatur besitzt das Sinterprodukt exakt die Ausgangsdimensionen des Formkörpers.
5. Auf einer Glasplatte wird eine Pulvermischung aus 15 % Titanpulver, 50 % Titanhydrid, 20 % Magnesiumoxid und 15 % Titanoxid mit Wasser und Polyäthylenglykol zu einer homogenen Paste vermischt und unter Zuhilfenahme von geeigneten Formen der gewünschte Formkörper z.B. Inlays, Onlays, Kronen, hergestellt.
   Nach dem Trocknen und Abbinden wir in einem programmgesteuerten Brennprozess mit einer Aufheizrate von ca. 2^{o} C/min gebrannt, wobei zunächst das beim Abbindeprozeß eingebaute Wasser entweicht. Im weiteren Verlauf wird das Polyäthylenglykol oxidiert und die hierdurch gebildeten Gase ausgetrieben. Es entsteht ein formstabiler Rohling mit einer Gründichte von ca. 68 Vol. %. Beim weiteren Erhitzen werden die noch vorhandenen Poren durch das sich aus dem Titan und dem Titanhydrid bildende Titanoxid allmählich geschlossen. Bei einer Temperatur von ca. 1300^{o} C wird der Formkörper während 3 Stunden auf seine Enddichte gesintert. Nach Abkühlen auf Raumtemperatur besitzt das Sinterprodukt exakt die Ausgangsdimensionen des Formkörpers.
6. In einem Kneter werden folgende Rohstoffe homogen zu einer plastischen Masse vermischt:
   50 % Titanpulver, mittlere Teilchengröße 7 µm,
   17 % Titansuboxid, mittlere Teilchengröße 15 µm (Ti₂0₃),
   10 % Magnesiumoxid, mittlere Teilchengröße 8µm,
   23 % Methacrylat.
   Aus der so hergestellten Masse wird unter Zuhilfenahme von geeigneten Formen der gewünschte Formkörper z.B. Inlays, Onlays, Kronen, hergestellt. Nach dem Aushärten des Binders wird in einem programmgesteuerten Brennprozess analog Beispiel 1 zunächst das Methacrylat ausgebrannt. Es entsteht ein formstabiler Rohling mit einer Gründichte von ca. 68 % Vol. %. Bei weiteren Erhitzen werden die noch vorhandenen Poren durch das sich bildende Titandioxid allmählich geschlossen. Bei einer Temperatur von ca. 1300^{o} C wird der Formkörper während 3 Stunden auf seine Enddichte gesintert. Nach Abkühlen auf Raumtemperatur besitzt das Sinterprodukt exakt die Ausgangsdimensionen des Formkörpers.
7. In einem Kneter werden folgende Rohstoffe bei ca. 160 ^{o}C homogen zu einer plastischen Masse vermischt:
   40 % Titanpulver, mittlere Teilchengröße 7µm,
   22% Magnesiumtitanat, mittlere Teilchengröße 2 µm,
   28 % Aluminiumpulver, mittlere Teilchengröße 13 µm,
   10 % Wachs.
   Aus der so hergestellten, bei höherer Temperatur plastischen Masse, wird unter Zuhilfenahme von geeigneten Formen der gewünschte Formkörper z.B. Inlays, Onlays, Kronen, hergestellt. In einem programmgesteuerten Brennprozess wird zunächst mit einer Aufheizrate von ca. 0,5 ^{o}C/min bis zu einer Temperatur von 370^{o} C das Wachs ausgebrannt. Es entsteht ein formstabiler Rohling mit einer Gründichte von ca. 67 Vol. %. Beim weiteren Erhitzen mit einer Aufheizrate von ca. 2^{o} C/min auf ca. 1500^{o} C werden die noch vorhandenen Poren durch das sich bildende Titanoxid und Aluminiumoxid allmählich geschlossen. Bei einer Temperatur von ca. 1500^{o} C wird während 3 Stunden der Formkörper auf seine Enddichte gesintert. Nach Abkühlen auf Raumtemperatur besitzt das Sinterprodukt exakt die Ausgangsdimensionen des Formkörpers.

## Patentansprüche

1. Verfahren zur Herstellung oxidkeramischer Zahnersatzstücke, wie Inlays, Kronen oder sonstiger Prothesenteile, in einem Arbeitsgang, durch Sintern eines Gemisches aus Oxidkeramikteilchen mit Bindemitteln und/oder Anmischflüssigkeiten,
**dadurch gekennzeichnet,**
daß den Keramikteilchen Teilchen aus leicht oxidierbaren Metallen, Metallsuboxiden und/oder Metallhydriden zugesetzt werden, wobei deren stabile Oxide eine zahnähnliche Färbung aufweisen müssen,
daß aus dem Gemisch Grünlinge mit den gewünschten Enddimensionen geformt werden,
daß das Sintern unter Luft- oder Sauerstoffzufuhr erfolgt, und
daß das Gewichtsverhältnis von Metall-,
Metallsuboxid- und/oder Metallhydridteilchen zu Keramikteilchen so gewählt wird, daß die Volumenschrumpfung der Keramikteilchen beim Sintern durch die Volumenvergrößerung der Metall-, Metallsuboxid- und/oder Metallhydridteilchen bei der Oxidation kompensiert wird.

2. Verfahren nach Anspruch 1,
**dadurch gekennzeichnet,**
daß als Metallteilchen Pulver aus Titan, Zirkonium und/oder Aluminium verwendet werden.
